# EUROPEAN PATENT APPLICATION

(11) **EP 3 293 252 A1**
(43) Date of publication of application: **14.03.2018**
(21) Application number: 17189060.1
(22) Date of filing: 01.09.2017
(51) Int. Cl.: C12M 1/00, C12M 1/26, C12M 1/02, C12M 1/34

(54) **BUFFER TANK AND CULTURE SYSTEM**

(30) Priority: 08.09.2016 JP 2016175829
(71) Applicant: Tokyo Electron Limited, Tokyo 107-6325 (JP)
(72) Inventor: Hatabayashi, Kunitada, Minato-ku, Tokyo 107-6325 (JP); Kagawa, Kenichi, Minato-ku, Tokyo 107-6325 (JP); Kitahara, Toshifumi, Minato-ku, Tokyo 107-6325 (JP); Yoda, Yusuke, Minato-ku, Tokyo 107-6325 (JP)
(74) Representative: Diehl & Partner GbR

(57) **Abstract**

A buffer tank (30) for storing a culture medium for cell culture, includes: a tank main body (32) including a cylindrical inner surface (33) extending in a vertical direction, an inverted conical storage bottom surface (34) formed below the inner surface (33), and a storage space (31) defined by the inner surface (33) and the inverted conical storage bottom surface (34) and configured to store the culture medium; an injection portion (40) configured to inject the culture medium from the inverted conical storage bottom surface (34) of the tank main body (32) into the storage space (31); and a discharge portion (44) configured to discharge the culture medium stored in the storage space (31). The injection portion (40) injects the culture medium toward a central axis line (X) of the inner surface (33) of the tank main body (32) and obliquely upward with respect to the central axis line (X).

## Description

### TECHNICAL FIELD

The present disclosure relates to a buffer tank and a culture system.

### BACKGROUND

In recent years, research and development of regenerative medicine that artificially creates a target tissue or organ by cell culture is underway. In order to carry out a culturing operation of cells and the like, a culture system satisfying a predetermined standard, for example, GMP (Good Manufacturing Practice) is used.

In such a culture system, to prevent gradual deterioration of a culture environment in a culture container requires periodically replacing a liquid culture medium (also referred to as a culture solution) inside the culture container. At this time, by supplying a fresh culture medium to the culture container, an old culture medium inside the culture container is extruded and discharged from the culture container. The old culture medium thus discharged is supplied to (sampled by) a culture medium analysis part where component analysis is performed to confirm whether or not the cell culture inside the culture container has been appropriately performed.

However, there may be a case where bias occurs in the components of the culture medium stored in the culture container. This may degrade the accuracy of component analysis. In order to cope with this, it is effective to homogenize the discharged culture medium before supplying it to the culture medium analysis part.

Incidentally, there is known a tank for stirring a diluted cell suspension mixed with a culture medium to resolve non-uniformity of cell distribution. In such a tank, by repeating an operation of aspirating a portion of the cell suspension inside the tank from the tank and an operation of injecting the same amount of the cell suspension into the tank, a stirring flow is generated in the cell suspension inside the tank.

However, there is a problem that time is consumed to stir the cell suspension in the conventional tank. For example, in a case where the tank is used for homogenization of the culture medium discharged from the culture container, a pH, dissolved oxygen concentration, carbon dioxide concentration and the like of the culture medium may be changed while stirring. In this case, a problem is posed in that the accuracy of component analysis of the culture medium decreases and the quality of the cultured cells declines.

Further, for example, in a case where the conventional tank is used as a tank for storing a fresh culture medium to remove air bubbles in the fresh culture medium before supplying the fresh culture medium to the culture container, a period of time is taken until the heated culture medium is homogenized. Thus, the culture medium may be degraded. Even in this case, there arises a problem that the quality of the cultured cells declines. The same problem may arise when additives are added to a culture medium inside the tank.

### SUMMARY

Some embodiments of the present disclosure provide a buffer tank and a culture system capable of shortening a period of time taken from when a culture medium is injected till when the culture medium is homogenized and improving the quality of cultured cells.

According to one embodiment of the present disclosure, there is provided a buffer tank for storing a culture medium for cell culture, includes: a tank main body including a cylindrical inner surface extending in a vertical direction, an inverted conical storage bottom surface formed below the cylindrical inner surface, and a storage space defined by the cylindrical inner surface and the inverted conical storage bottom surface and configured to store the culture medium; an injection portion configured to inject the culture medium from the inverted conical storage bottom surface of the tank main body into the storage space; and a discharge portion configured to discharge the culture medium stored in the storage space, wherein the injection portion is configured to inject the culture medium toward a central axis line of the cylindrical inner surface of the tank main body and obliquely upward with respect to the central axis line.

According to another embodiment of the present disclosure, there is provided a culture system, which includes: a container holding part configured to hold a culture container; a culture medium analysis part configured to analyze a culture medium discharged from the culture container held by the container holding part; and the aforementioned buffer tank installed between the container holding part and the culture medium analysis part and configured to store the culture medium discharged from the culture container held by the container holding part.

According to yet another embodiment of the present disclosure, there is provided a culture system, which includes: a culture medium supply source configured to supply a fresh culture medium; a container holding part configured to hold a culture container; and the aforementioned buffer tank installed between the culture medium supply source and the container holding part and configured to store the fresh culture medium supplied from the culture medium supply source.

### BRIEF DESCRIPTION OF THE DRAWINGS

The accompanying drawings, which are incorporated in and constitute a part of the specification, illustrate embodiments of the present disclosure, and together with the general description given above and the detailed description of the embodiments given below, serve to explain the principles of the present disclosure.
FIG. 1 is a view showing a schematic configuration of a culture system according to the present embodiment.
FIG. 2 is a plan view showing a culture container shown in FIG. 1.
FIG. 3 is a sectional view showing the culture container shown in FIG. 2.
FIG. 4 is a vertical sectional view showing a buffer tank for analysis shown in FIG. 1.
FIG. 5 is a horizontal sectional view showing an injection portion of the buffer tank for analysis shown in FIG. 4.
FIG. 6 is a horizontal sectional view showing an analyzer of the buffer tank for analysis shown in FIG. 4.
FIG. 7 is a view showing a first modification of FIG. 1 and is a partial diagram showing a part of a schematic configuration of a culture system.

### DETAILED DESCRIPTION

Hereinafter, one embodiment of the present disclosure will be described with reference to the drawings. In the drawings attached hereto, for the sake of ease of understanding of the drawings, the scales, the aspect ratios and the like are changed and exaggerated from those of the actual ones. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, systems, and components have not been described in detail so as not to unnecessarily obscure aspects of the various embodiments.

A culture system according to the present embodiment may be used for culturing all kinds of cells and may be used in culturing various cells including pluripotent stem cells such as (human) iPS cells, (human) ES cells or the like, chondrocytes such as bone marrow stromal cells (MSCs) or the like, dendritic cells, and so forth. In the present embodiment, hereinafter, description will be made mainly assuming the use for culturing iPS cells. However, this is nothing more than an example.

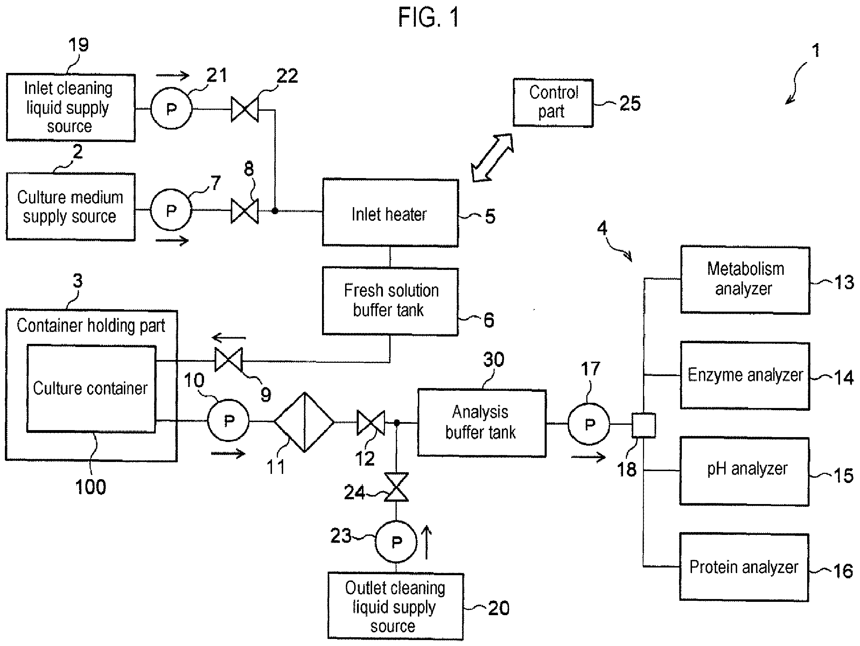

First, a schematic configuration of a culture system according to an embodiment of the present disclosure will be described with reference to FIG. 1.

As shown in FIG. 1, the culture system 1 includes a culture medium supply source 2 configured to supply a fresh culture medium, a container holding part 3 capable of holding a culture container 100 for culturing cells, and a culture medium analysis part 4 configured to analyze components of the culture medium that is extracted from the culture container 100 held by the container holding part 3.

The culture medium supply source 2 stores a fresh culture medium for cell culture to be supplied to the culture container 100. The culture medium supply source 2 is installed inside, for example, a refrigerator. At the time of storage, the culture medium is stored at a low temperature (for example, about 4 degrees C) to prevent deterioration of components.

An inlet heater 5 and a fresh solution buffer tank 6 are provided in this order between the culture medium supply source 2 and the container holding part 3.

The inlet heater 5 heats the culture medium supplied from the culture medium supply source 2 to set a temperature of the culture medium to a high temperature (for example, about 37 degrees C). The culture medium thus heated is discharged from the inlet heater 5 and supplied to the fresh solution buffer tank 6. An inlet pump 7 for supplying a culture medium from the culture medium supply source 2 to the inlet heater 5 is installed between the culture medium supply source 2 and the inlet heater 5. The pump for supplying the culture medium is not limited to being constituted by the inlet pump 7. For example, a common pump (not shown) may be installed at the downstream side of the junction of a line from the culture medium supply source 2 and a line from an inlet cleaning liquid supply source 19 and at the upstream side of the inlet heater 5. The common pump may have a culture medium supply function. In this case, the common pump may also have a cleaning liquid supply function. Thus, an inlet cleaning pump 21 to be described later may be omitted.

The fresh solution buffer tank 6 stores the culture medium heated by the inlet heater 5 and removes air bubbles in the culture medium. The fresh solution buffer tank 6 has an internal space for storing the culture medium and a vent portion (both not shown). The internal space of the fresh solution buffer tank 6 communicates with the surrounding atmosphere of the fresh solution buffer tank 6 (an internal clean atmosphere of a chamber of the culture system 1) via the vent portion. As a result, when air bubbles are mixed in the culture medium stored in the fresh solution buffer tank 6, the air bubbles float up and are removed from the culture medium. That is to say, since the dissolved gas and minute air bubbles contained in the fresh culture medium develop and expand due to the high temperature, it is possible to efficiently remove air bubbles from the culture medium stored in the internal space. Further, by having the vent portion, it is possible to smoothly supply and discharge the culture medium to and from the fresh solution buffer tank 6. A vent filter (not shown) is installed in the vent portion to prevent infiltration of foreign matters into the internal space of the fresh solution buffer tank 6.

A culture medium storage capacity of the internal space of the fresh solution buffer tank 6 may be set larger than that of the culture container 100 so that when replacing the culture medium in the culture container 100, the old culture medium in the culture container 100 can be extruded and completely replaced with a fresh one. For example, when the capacity of the culture container 100 is 18 mL, the culture medium storage capacity of the fresh solution buffer tank 6 may be set to, for example, 30 mL, which is larger than the capacity of the culture container 100.

A first inlet opening/closing valve 8 is installed between the inlet pump 7 and the inlet heater 5. The first inlet opening/closing valve 8 controls the supply of the culture medium from the culture medium supply source 2 to the fresh solution buffer tank 6. A second inlet opening/closing valve 9 is installed between the fresh solution buffer tank 6 and the container holding part 3 to control the supply of the culture medium from the fresh solution buffer tank 6 to the culture container 100. The fresh solution buffer tank 6 is disposed at a position higher than the container holding part 3. This makes it easy to supply the culture medium from the fresh solution buffer tank 6 to the culture container 100 held in the container holding part 3.

The container holding part 3 is configured to hold the culture container 100. In a case where the culture system 1 incorporates an incubator (not shown) for performing cell culture, the incubator is disposed inside the chamber of the culture system 1 described above. The container holding part 3 is installed inside a housing of the incubator. The housing of the incubator is configured to adjust at least one of the temperature, the humidity, and the gas concentration of the internal atmosphere. For example, the temperature of the internal atmosphere of the housing is adjusted so that the temperature of the culture container 100 held in the container holding part 3 becomes about 37 degrees C. On the other hand, in a case where the culture system 1 and the incubator are installed apart from each other, the container holding part 3 serves to temporarily hold the culture container 100 transferred from the incubator to the container holding part 3 in order to replace the culture medium. In this case, the culture container 100 held in the container holding part 3 is accommodated in, for example, a housing for maintaining a sterilization space in the chamber of the culture system 1. Details of the culture container 100 will be described later.

An analysis buffer tank 30 is installed between the container holding part 3 and the culture medium analysis part 4. The analysis buffer tank 30 stores a certain amount of the culture medium discharged from the culture container 100. The culture medium is mixed and stored in the analysis buffer tank 30. Thus, the culture medium stored in the analysis buffer tank 30 is homogenized. A culture medium storage capacity of the analysis buffer tank 30 may be set smaller than that of the culture container 100. Details of the analysis buffer tank 30 will be described later.

Between the container holding part 3 and the analysis buffer tank 30, a first outlet pump 10, a culture medium filter 11 and an outlet opening/closing valve 12 are installed in this order. Among these, the first outlet pump 10 draws out the culture medium after cell culture from the culture container 100 and supplies the same to the analysis buffer tank 30. At this time, simultaneously with the draw-out of the culture medium from the culture container 100, the supply of a fresh culture medium from the fresh solution buffer tank 6 to the culture container 100 is prompted. As a result, a fresh culture medium is supplied to the culture container 100 so that the culture medium replacement is performed. The culture medium filter 11 is configured to remove, from the culture medium, solid matters (e.g., cultured cells, etc.) contained in the culture medium discharged from the culture container 100. The outlet opening/closing valve 12 is configured to control the supply of the culture medium from the culture container 100 to the analysis buffer tank 30.

The culture medium analysis part 4 according to the present embodiment includes a metabolism analyzer 13, an enzyme analyzer 14, a pH analyzer 15, and a protein analyzer 16. The metabolism analyzer 13 analyzes the concentration and component of a metabolite contained in the culture medium discharged from the culture container 100. The enzyme analyzer 14 analyzes the concentration and component of an enzyme contained in the culture medium. The pH analyzer 15 analyzes the pH of the culture medium. The protein analyzer 16 analyzes the concentration and component of a protein contained in the culture medium. The analysis of the culture medium performed in these analyzers 13 to 16 is carried out by a destructive inspection method. The culture medium used for the analysis is discarded without being reused.

Between the analysis buffer tank 30 and the culture medium analysis part 4, a second outlet pump 17 and an outlet switching valve 18 are installed in this order. Among these, the second outlet pump 17 is configured to supply the culture medium from the analysis buffer tank 30 to the culture medium analysis part 4. The outlet switching valve 18 is configured to switch flow paths so that culture medium discharged from the analysis buffer tank 30 is selectively supplied to any one of the metabolism analyzer 13, the enzyme analyzer 14, the pH analyzer 15 and the protein analyzer 16. In this way, the culture medium supplied from the analysis buffer tank 30 can be separately supplied to these four analyzers 13 to 16.

The culture system 1 according to the present embodiment further includes an inlet cleaning liquid supply source 19 and an outlet cleaning liquid supply source 20. Of these, the inlet cleaning liquid supply source 19 supplies a cleaning liquid to the inlet heater 5 to clean the inlet heater 5 and the fresh solution buffer tank 6. The cleaning liquid supplied to the inlet heater 5 is supplied to the fresh solution buffer tank 6, whereby the inlet heater 5 and the fresh solution buffer tank 6 are cleaned. Between the inlet cleaning liquid supply source 19 and the inlet heater 5, an inlet cleaning pump 21 and an inlet cleaning opening/closing valve 22 are installed in this order to control the supply of the cleaning liquid from the inlet cleaning liquid supply source 19 to the inlet heater 5. The outlet cleaning liquid supply source 20 supplies the cleaning liquid to the analysis buffer tank 30 to clean the analysis buffer tank 30. Between the outlet cleaning liquid supply source 20 and the analysis buffer tank 30, an outlet cleaning pump 23 and an outlet cleaning opening/closing valve 24 are installed in this order to control the supply of the cleaning liquid from the outlet cleaning liquid supply source 20 to the analysis buffer tank 30.

As shown in FIG. 1, the culture system 1 further includes a control part 25. The control part 25 is configured to control the opening/closing valves and the pumps described above.

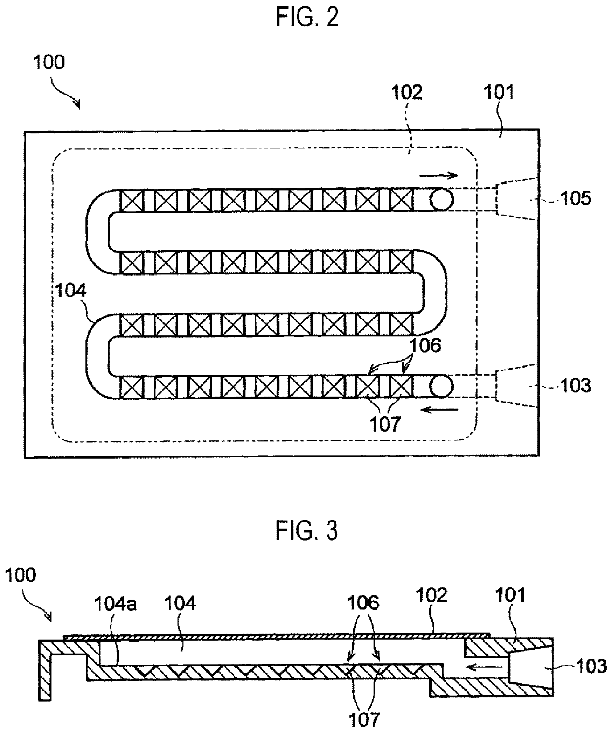

Next, the culture container 100 according to the present embodiment will be described with reference to FIGS. 2 and 3.

As shown in FIGS. 2 and 3, the culture container 100 includes a container body 101 and a flat plate 102 attached onto one surface of the container body 101. The container body 101 includes an inflow port 103 into which a culture medium (a suspension in which cells are dispersed, a release agent, a phosphate buffered saline (PBS), etc., as well as the culture medium) is introduced, a passage 104 through which the culture medium introduced from the inflow port 103 passes, and an outflow port 105 from which the culture medium passed through the passage 104 is discharged. Of these, the inflow port 103 is connected to the above-described fresh solution buffer tank 6, and the outflow port 105 is connected to the analysis buffer tank 30.

The passage 104 of the container body 101 is formed in a groove shape in the one surface onto which the flat plate 102 of the container body 101 is attached. The diameter of the passage 104 (namely, the depth and width of the groove) is, for example, 2 to 4 mm. In addition, the passage 104 of the container body 101 has a serpentine portion in a plan view, namely a portion in which linear portions and folded portions are alternately connected. As a result, the total length of the passage 104 is extended without enlarging the container body 101, whereby the passage 104 having an elongated shape is formed.

As shown in FIGS. 2 and 3, in a passage bottom surface 104a of the passage 104, a plurality of cell seeding regions 106 in which cells passing through the passage 104 are seeded is formed side-by-side along the passage 104. In the present embodiment, in the passage bottom surface 104a of the passage 104, recesses 107 are formed in a concentric relationship with the cell seeding regions 106.

When replacing the culture medium in the culture container 100, the first outlet pump 10 is driven so that the old culture medium existing in the passage 104 is drawn out and discharged from the outflow port 105. Concurrently, a fresh culture medium supplied from the fresh solution buffer tank 6 is introduced into the passage 104 from the inflow port 103. During that time, the old culture medium existing in the passage 104 is extruded by the fresh culture medium and discharged from the outflow port 105. In this case, the fresh culture medium and the old culture medium flow along the passage 104. It is therefore possible to prevent the fresh culture medium and the old culture medium from being mixed with each other and to easily replace the old culture medium with the fresh culture medium.

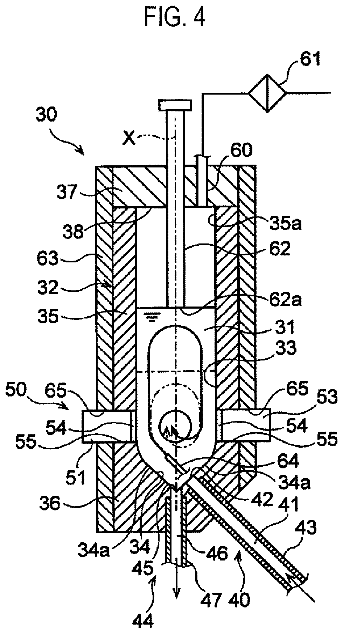

Next, the buffer tank according to this embodiment will be described with reference to FIGS. 4 to 6. In the present embodiment, description will be made on an example in which the buffer tank according to the present disclosure is applied to the analysis buffer tank 30 shown in FIG. 1.

As shown in FIG. 4, the analysis buffer tank 30 includes a tank main body 32 having a storage space 31 for storing the culture medium. The tank main body 32 may be formed of an arbitrary material such as a resin material or a metal material. In the case where the tank main body 32 is made of a material having good thermal conductivity such as a metal material or the like, it is possible to efficiently transfer heat generated by a silicone rubber heater 63 (to be described later) to the culture medium, thereby shortening the heating time of the culture medium. Examples of the metal material include stainless steel and aluminum alloy.

The storage space 31 of the tank main body 32 is defined by a cylindrical inner surface 33 extending in the vertical direction, and a storage bottom surface 34 having an inverted conical shape and formed below the inner surface 33. The inner surface 33 and the storage bottom surface 34 are smoothly connected in a curved shape. More specifically, the tank main body 32 includes a cylindrical tank body portion 35 and a tank bottom portion 36 installed below the tank body portion 35. Of these, the inner surface 33 is formed by an inner surface of the tank body portion 35, and the storage bottom surface 34 having an inverted conical shape (downwardly tapered conical shape) is formed by an upper surface of the tank bottom portion 36. There is shown an example in which the apex angle of the storage bottom surface 34 is about 90 degrees in FIG. 4. However, the present disclosure is not limited thereto. In some embodiments, a water repellent coating may be applied to the inner surface 33 and the storage bottom surface 34. In this case, when the culture medium stored in the storage space 31 is discharged, it is possible to prevent the culture medium from remaining on the inner surface 33 or the storage bottom surface 34 due to the surface tension.

In the present embodiment, a lid portion 37 is installed above the tank body portion 35. The lid portion 37 is detachably attached to an upper end of the tank body portion 35 by a bolt (not shown) or the like. A lower surface 38 of the lid portion 37 defines the storage space 31. The lid portion 37 is configured to close an upper end opening 35a of the tank body portion 35. The lid portion 37 may be formed of an arbitrary material such as a resin material or a metal material. As an example, the lid portion 37 may be formed of the resin material, for example, PEEK (polyetheretherketone).

An injection portion 40 is installed in the tank bottom portion 36. The injection portion 40 is configured to inject the culture medium discharged from the culture container 100 into the storage space 31 through the storage bottom surface 34 of the tank main body 32. The injection portion 40 injects the culture medium toward a central axis line X of the inner surface 33 of the tank main body 32 and obliquely upward with respect to the central axis line X. In the present embodiment, when viewing the storage bottom surface 34 as a pair of generatrices 34a in a cross section including the central axis line X and the injection portion 40, the culture medium is injected in a direction parallel to the generatrix 34a located at the side opposite to the generatrix 34a existing at the side where the injection portion 40 is installed. As shown in FIG. 4, when the apex angle of the storage bottom surface 34 is 90 degrees, in the sectional view, the culture medium is injected in a direction perpendicular to the storage bottom surface 34 of the tank main body 32 (the generatrix 34a existing at the side where the injection portion 40 is installed).

More specifically, the injection portion 40 includes an injection path 41 penetrating the tank bottom portion 36 and an injection opening 42 formed at the downstream end portion of the injection path 41. The injection opening 42 means an opening formed in the storage bottom surface 34 of the tank main body 32.

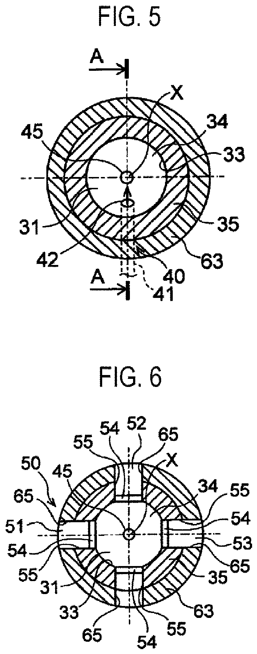

As shown in FIG. 5, the injection path 41 extends along a plane including the central axis line X and the injection opening 42. In other words, the injection path 41 extends in the radial direction around the central axis line X as viewed from above. The plane including the central axis line X and the injection opening 42 refers to a plane A-A shown in FIG. 5 when viewed from above. FIG. 4 corresponds to a cross section taken along line A-A. In addition, when viewed in a cross section on the plane including the central axis line X and the injection opening 42 (namely, when viewed in the cross section shown in FIG. 4), the injection path 41 extends in a direction parallel to the generatrix 34a existing at the side where the injection opening 42 of the tank main body 32 is not formed (in a direction perpendicular to the generatrix 34a existing at the side where the injection opening 42 is formed). The injection path 41 is connected to the above-mentioned outlet opening/closing valve 12. The culture medium discharged from the culture container 100 is supplied to the injection path 41 through the outlet opening/closing valve 12. In the embodiment shown in FIGS. 4 and 5, an injection plug 43 is attached to the tank bottom portion 36. The injection path 41 is formed inside the injection plug 43.

A flow rate of the culture medium injected from the injection portion 40 into the storage space 31 is not particularly limited as long as it is possible to homogenize the culture medium stored in the storage space 31 by forming a vortex *(see* FIG. 4) which will be described later. For example, when the diameter of the injection opening 42 is 1 mm, the flow rate of the culture medium may fall within a range of 20 to 30 mL/min. By setting the flow rate to 20 mL/min or more, a more suitable vortex can be formed by the culture medium inside the storage space 31. By setting the flow rate to 30 mL/min or less, it is possible to prevent the energy of the outlet pump from being wasted by the pressure loss in the culture medium filter 11 or the like.

As shown in FIG. 4, a discharge portion 44 is formed in the tank bottom portion 36. The discharge portion 44 discharges the culture medium stored in the storage space 31. The discharge portion 44 includes a discharge opening 45 formed at the lowermost point of the storage bottom surface 34 of the tank main body 32 and a discharge path 46 extending downward from the discharge opening 45. Among these, the discharge opening 45 means an opening formed in the storage bottom surface 34 of the tank main body 32. The discharge opening 45 is disposed so as to overlap with the central axis line X when viewed from above *(see* FIGS. 5 and 6). The discharge path 46 penetrates the tank bottom portion 36 and is connected to the above-mentioned outlet pump. In the embodiment shown in FIG. 4, a discharge plug 47 is attached to the tank bottom portion 36. The discharge path 46 is formed inside the discharge plug 47.

As shown in FIG. 4, a tank analyzer 50 configured to analyze components of the culture medium stored in the storage space 31 is installed in the tank main body 32. In this embodiment, as shown in FIG. 6, the tank analyzer 50 includes a pH measurement sensor 51 configured to measure pH of the culture medium, an oxygen concentration sensor 52 configured to measure a dissolved oxygen concentration in the culture medium, and a carbon dioxide concentration sensor 53 configured to measure a carbon dioxide concentration in the culture medium. Among these, by measuring the pH of the culture medium using the pH measurement sensor 51, it is possible to confirm whether the pH of the culture medium falls within an appropriate range. By measuring the dissolved oxygen concentration in the culture medium using the oxygen concentration sensor 52, it is possible to confirm the amount of oxygen used in the cells under culture. By measuring the carbon dioxide concentration in the culture medium using the carbon dioxide concentration sensor 53, it is possible to confirm an activity state of the cells under culture. The components of the culture medium measured by these sensors 51 to 53 are used for comparative evaluation. That is to say, the components of the culture medium measured by these sensors 51 to 53 are used to compare and evaluate the components of the culture medium measured up to the previous time and the components of the culture medium measured at the current time.

The pH measurement sensor 51 is not particularly limited and may be, for example, an optical sensor. The optical sensor may be, for example, a sensor configured to detect fluorescence emitted from a fluorescent chip disposed in the storage space 31 at a light detection end of the sensor and to analyze pH from the fluorescence component thus detected. A similar optical sensor may also be applied to the oxygen concentration sensor 52 and the carbon dioxide concentration sensor 53. In the case of using such an optical sensor, unlike the respective analyzers 13 to 16 of the culture medium analysis part 4 shown in FIG. 1, the components of the culture medium can be analyzed by a non-destructive inspection method, and the culture medium used for the analysis can be used for the analysis in the culture medium analysis part 4.

As shown in FIGS. 4 and 6, the respective sensors 51 to 53 are disposed in window portions 54 installed in the inner surface 33 of the tank main body 32. That is to say, in the present embodiment, as shown in FIG. 6, four sensor insertion holes 55 penetrating the tank body portion 35 in the direction orthogonal to the central axis line X are formed in the tank main body 32, and the window portions 54 are installed at respective inner end portions of the sensor insertion holes 55. The four window portions 54 are arranged at equal intervals in the circumferential direction of the tank body portion 35 when viewed from above and are arranged at a pitch of 90 degrees. Three sensors 51 to 53 are separately disposed in different window portions 54 among the four window portions 54. When the sensors 51 to 53 are the optical sensors mentioned above, the window portions 54 have a light transmitting property, the above-described fluorescent chip is disposed in each of the window portions 54 at the side of the inner surface 33 of the tank main body 32, and the light detection end of each of the optical sensors is disposed in each of the sensor insertion holes 55. Another sensor may be arranged in the remaining one window portion 54. As a reserve, a sensor may not be arranged in the remaining one window portion 54.

The above-described four window portions 54 are disposed below the center of the tank body portion 35 in the vertical direction. In the embodiment shown in FIG. 4, the four window portions 54 are disposed at a lower end portion of the tank body portion 35. Thus, the respective sensors 51 to 53 of the tank analyzer 50 can be disposed in the lower region of the storage space 31. In this case, it is possible to reduce the amount of a buffer solution used for preserving the respective sensors 51 to 53 while not performing the analysis of the culture medium.

As shown in FIG. 4, a vent portion 60 is installed in the lid portion 37. The vent portion 60 brings the storage space 31 of the analysis buffer tank 30 into communication with the ambient atmosphere of the analysis buffer tank 30 (the internal clean atmosphere of the chamber of the culture system 1 described above). The vent portion 60 is configured to smoothly perform the supply and discharge of the culture medium to and from the analysis buffer tank 30. A vent filter 61 is installed in the vent portion 60 to prevent entry of foreign matters into the storage space 31.

A liquid level sensor 62 configured to detect a liquid level of the culture medium stored in the storage space 31 is installed in the lid portion 37. The control part 25 shown in FIG. 1 is connected to the liquid level sensor 62. The control part 25 controls the outlet opening/closing valve 12 based on the liquid level of the culture medium measured by the liquid level sensor 62. That is to say, when the liquid level of the culture medium is detected by the liquid level sensor 62, the outlet opening/closing valve 12 is closed to stop the supply of the culture medium to the analysis buffer tank 30. Depending on a position of a detection part 62a of the liquid level sensor 62, the culture medium storage capacity of the analysis buffer tank 30 is set. The culture medium storage capacity of the analysis buffer tank 30 is set to be smaller than the capacity of the culture container 100 as described above. The remainder of the old culture medium discharged from the culture container 100 is discharged via a discharge line not shown. As a result, a portion of the old culture medium, after cell culture in the culture container 100, is collected in the storage space 31 of the analysis buffer tank 30. The collection amount thereof is constant each time.

As shown in FIG. 4, a silicone rubber heater 63 (used as a heating part) is installed around the tank main body 32 and the lid portion 37. The silicon rubber heater 63 is installed so as to cover outer peripheral surfaces of the tank main body 32 and the lid portion 37. The silicon rubber heater 63 is configured to heat the tank main body 32 and the lid portion 37 and to thermally insulate the tank main body 32 and the lid portion 37 from the surrounding atmosphere. A temperature sensor 64 configured to measure a temperature of the culture medium stored in the storage space 31 is installed in the tank bottom portion 36 of the tank main body 32. The temperature sensor 64 is attached to the storage bottom surface 34 of the tank main body 32. The control part 25 shown in FIG. 1 is connected to the temperature sensor 64. The control part 25 controls ON/OFF of the silicon rubber heater 63 based on the temperature of the culture medium measured by the temperature sensor 64. That is to say, when the temperature of the culture medium is lower than a predetermined temperature (for example, 37 degrees C), the silicone rubber heater 63 is turned on. When the temperature of the culture medium is equal to or higher than the predetermined temperature, the silicon rubber heater 63 is turned off. Second sensor insertion holes 65 communicating with the respective sensor insertion holes 55 are formed in the silicone rubber heater 63 so that the light detection ends of the above-described sensors 51 to 53 can be respectively inserted into the second sensor insertion holes 65. Instead of the silicon rubber heater 63, a temperature controller capable of not only heating but also cooling the tank main body 32 and the lid portion 37 may be used.

Next, the operation of the present embodiment having such a configuration will be described.

When sampling the old culture medium at the time of replacing the culture medium in the culture container 100, the first outlet pump 10 shown in FIG. 1 is first driven, and the second inlet opening/closing valve 9 and the outlet opening/closing valve 12 are opened. As a result, the old culture medium is drawn out from the culture container 100 and supplied to the injection portion 40 *(see* FIG. 4) of the analysis buffer tank 30. At this time, the fresh culture medium stored in the fresh solution buffer tank 6 is introduced into the passage 104 via the inflow port 103 *(see* FIGS. 2 and 3) of the culture container 100. The old culture medium in the passage 104 is extruded and discharged by the fresh culture medium.

The old culture medium supplied to the injection portion 40 of the analysis buffer tank 30 is injected into the storage space 31 from the injection opening 42 through the injection path 41 as shown in FIG. 4.

At this time, the injection path 41 extends along the plane (the surface indicated by the line A-A in FIG. 5) including the central axis line X and the injection opening 42, and extends in a direction parallel to the generatrix 34a at the side where the injection opening 42 shown in FIG. 4 is not formed. Thus, the culture medium that passed through the injection opening 42 is injected in the direction extending obliquely upward with respect to the central axis line X toward the central axis line X and in the direction parallel to the generatrix 34a. As a result, the culture medium is injected into the storage space 31 while forming a vortex *(see* the two-dot chain line arrow in FIG. 4) which swirls in the vertical direction and moves along the plane including the central axis line X and the injection opening 42. The vortex thus formed swirls so as to stride over the central axis line X along the plane, and involves the culture medium near the storage bottom surface 34 of the storage space 31 and the culture medium near the liquid surface. The vortex may be formed over the entire culture medium inside the storage space 31. At this time, it is possible to suppress formation of stagnation, which is hard to be caught in the vortex, in the culture medium inside the storage space 31.

As the injection of the culture medium continues, the liquid level of the culture medium stored in the storage space 31 rises. Even while the liquid level of the medium is rising, the culture medium is injected obliquely upward with respect to the central axis line X toward the central axis line X. For this reason, a vortex *(see* the solid line arrow in FIG. 4) similar to the vortex shown by the two-dot chain line in FIG. 4 and swirling in the vertical direction at a larger size is formed.

In this way, the culture medium inside the storage space 31 is mixed by the vortex. It is therefore possible to homogenize the culture medium inside the storage space 31. In particular, in this embodiment, as shown in FIG. 2, the passage 104 of the culture container 100 is formed in a serpentine elongated shape. Thus, there is a possibility that bias occurs in the components of the culture medium stored in the passage 104. In contrast, according to the analysis buffer tank 30 of the present embodiment, even for the culture medium discharged from the culture container 100 shown in FIG. 2, the culture medium can be quickly homogenized by the vortex formed at the time of injection and can be stored in the storage space 31.

When the liquid level of the culture medium inside the storage space 31 reaches the detection part 62a of the liquid level sensor 62, the liquid level of the culture medium is detected by the liquid level sensor 62. Then, the outlet opening/closing valve 12 is closed to stop the supply of the culture medium to the analysis buffer tank 30. In this case, a certain amount of the culture medium out of the total amount of the culture medium stored in the passage 104 of the culture container 100 is collected in the analysis buffer tank 30. As described above, the culture medium collected in the analysis buffer tank 30 is the culture medium stored in the same portion of the passage 104 each time. Therefore, even when bias occurs in the components of the culture medium stored in the passage 104 of the culture container 100, the components of the culture medium are biased in the same way each time. It is therefore possible to prevent the bias of the components of the culture medium from affecting the component analysis to be described later and to stabilize the component analysis.

After a predetermined time (for example, several minutes) from when the supply of the culture medium to the analysis buffer tank 30 is stopped, the components of the culture medium stored in the storage space 31 are analyzed by the tank analyzer 50 installed in the tank main body 32 through a nondestructive inspection method. In the present embodiment, the pH of the culture medium is measured by the pH measurement sensor 51, the dissolved oxygen concentration in the culture medium is measured by the oxygen concentration sensor 52, and the carbon dioxide concentration in the culture medium is measured by the carbon dioxide concentration sensor 53.

While the medium is stored in the storage space 31, if the temperature of the culture medium is lower than a predetermined temperature, the silicon rubber heater 63 is turned on, and the culture medium inside the storage space 31 is mainly heated via the tank main body 32. If the temperature of the culture medium reaches the predetermined temperature, the silicon rubber heater 63 is turned off. In this way, the temperature of the culture medium inside the storage space 31 is maintained at the predetermined temperature.

After the component analysis of the culture medium is completed, the culture medium is discharged from the storage space 31. In this case, the second outlet pump 17 is driven so that the culture medium inside the storage space 31 is discharged through the discharge opening 45 and the discharge path 46. The culture medium thus discharged is selectively supplied to any one of the metabolism analyzer 13, the enzyme analyzer 14, the pH analyzer 15 and the protein analyzer 16 of the culture medium analysis part 4 by the outlet switching valve 18.

When cleaning the storage space 31 of the analysis buffer tank 30, the outlet cleaning pump 23 is driven and the outlet cleaning valve 4 is opened. Thus, the cleaning liquid is supplied from the outlet cleaning liquid supply source 20 to the storage space 31. In this case, the outlet opening/closing valve 12 is closed. The cleaning liquid supplied to the storage space 31 can form a vortex *(see* FIG. 4) similar to that of the above-described culture medium. This makes it possible to increase the cleaning ability and to shorten the cleaning time of the storage space 31. In some embodiments, the cleaning liquid may be supplied up to a position higher than the detection part 62a of the liquid level sensor 62 in order to sufficiently clean the storage space 31. In a case where the cleaning liquid is discharged after cleaning, the cleaning liquid is discharged via a discharge line (not shown). By repeating the supply and discharge of the cleaning liquid a multiple number of times, it is possible to enhance the cleaning effect. The inlet heater 5 and the fresh solution buffer tank 6 may also be similarly cleaned by the cleaning liquid supplied from the inlet cleaning liquid supply source 19.

As described above, according to the present embodiment, the injection portion 40 for injecting the culture medium into the storage space 31 of the analysis buffer tank 30 is configured to inject the culture medium obliquely upward with respect to the central axis line X toward the central axis line X of the inner surface 33 of the tank main body 32. This makes it possible to form a vertically swirling vortex with respect to the culture medium stored in the storage space 31. It is also possible to homogenize the culture medium by mixing the stored culture medium while injecting the culture medium into the storage space 31. Therefore, it is possible to shorten a period of time taken from the start of injecting the culture medium into the storage space 31 to the homogenization of the culture medium stored in the storage space 31. In the case of analyzing the components of the culture medium thus homogenized, it is possible to suppress the change of the components of the culture medium and to improve the accuracy of component analysis of the culture medium. In this case, it is possible to improve the quality of the cultured cells.

Further, according to the present embodiment, when viewing the storage bottom surface 34 with reference to the pair of generatrices 34a in the cross section including the central axis line X and the injection portion 40, the culture medium is injected in the direction parallel to the generatrix 34a at the side opposite to the generatrix 34a existing at the side where the injection portion 40 is installed. As a result, it is possible to form a vortex that spans the entire culture medium inside the storage space 31. This makes it possible to further homogenize the culture medium inside the storage space 31.

Further, according to the present embodiment, the injection path 41 of the injection portion 40 extends along the plane including the central axis line X of the inner surface 33 of the tank main body 32 and the injection opening 42. As a result, the culture medium passed through the injection opening 42 can be injected obliquely upward with respect to the central axis line X toward the central axis line X.

Further, according to the present embodiment, the discharge opening 45 of the discharge portion 44 is formed at the lowermost position of the storage bottom surface 34 of the tank main body 32. Thus, when the culture medium inside the storage space 31 is discharged, it is possible to prevent the culture medium from remaining in the storage space 31.

Further, according to the present embodiment, the components of the culture medium inside the storage space 31 are analyzed by the tank analyzer 50. Thus, it is possible to analyze the components of the culture medium in advance before supplying the culture medium to the culture medium analysis part 4. In particular, according to the present embodiment, the tank analyzer 50 includes the pH measurement sensor 51, the oxygen concentration sensor 52, and the carbon dioxide concentration sensor 53. It is therefore possible to measure the pH of the culture medium inside the storage space 31, the dissolved oxygen concentration in the culture medium and the carbon dioxide concentration in the culture medium. In the present embodiment, the pH of the culture medium can be measured by the pH measurement sensor 51 of the tank analyzer 50 and the pH analyzer 15 of the culture medium analysis part 4. In this case, it is possible to improve the pH measurement accuracy of the culture medium. That is to say, for example, when the reliability of the absolute value of the pH measured by the pH analyzer 15 is good and the reproducibility of the pH measured by the pH measurement sensor 51 is good, the pH measurement data obtained from the pH measurement sensor 51 can be calibrated using the pH measurement data obtained from the pH analyzer 15. This makes it possible to improve the pH measurement accuracy of the culture medium. If there is no problem in the accuracy of the pH measurement data, the pH may be measured with only one of the pH measurement sensor 51 and the pH analyzer 15.

Further, according to the present embodiment, the tank main body 32 is heated by the silicon rubber heater 63 installed around the tank main body 32. As a result, the culture medium inside the storage space 31 can be heated by the silicon rubber heater 63 via the tank main body 32. Therefore, it is possible to maintain the culture medium inside the storage space 31 at a predetermined temperature, which suppresses the change in the components of the culture medium.

### (First Modification)

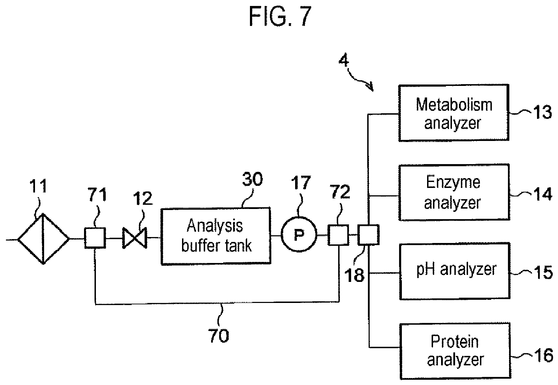

In the above-described embodiment, for example, as shown in FIG. 7, the culture medium stored in the storage space 31 of the analysis buffer tank 30 may be circulated by a circulation line 70. In the configuration shown in FIG. 7, a first circulation switching valve 71 is installed between the culture medium filter 11 and the outlet opening/closing valve 12, and a second circulation switching valve 72 is installed between the second outlet pump 17 and the outlet switching valve 18. A circulation line 70 connects the first circulation switching valve 71 and the second circulation switching valve 72. In FIG. 7, the outlet cleaning liquid supply source 20 is omitted for the clarity of the drawing.

In the configuration shown in FIG. 7, when circulating the culture medium inside the storage space 31, the first circulation switching valve 71 switches a flow path so that the culture medium flows from the circulation line 70 to the outlet opening/closing valve 12, and the second circulation switching valve 72 switches the flow path so that the culture medium flows from the second outlet pump 17 to the circulation line 70. Then, by opening the outlet opening/closing valve 12 and driving the second outlet pump 17, the culture medium inside the storage space 31 is discharged from the discharge portion 44 and introduced into the circulation line 70. The culture medium introduced into the circulation line 70 in this way is supplied to the injection portion 40 of the analysis buffer tank 30 and injected again into the storage space 31. In this way, it is possible to circulate the culture medium inside the storage space 31 and to further homogenize the culture medium.

In the configuration shown in FIG. 7, when the culture medium inside the culture container 100 is supplied to the analysis buffer tank 30, the first circulation switching valve 71 switches the flow path so that the culture medium flows from the culture medium filter 11 to the outlet opening/closing valve 12. When the culture medium inside the storage space 31 is supplied to the culture medium analysis part 4, the second circulation switching valve 72 switches the flow path so that the culture medium flows from the second outlet pump 17 to the culture medium analysis part 4.

### (Second Modification)

Further, in the above-described embodiment, there has been described an example in which the buffer tank according to the present disclosure is applied to the analysis buffer tank 30 shown in FIG. 1. However, the present disclosure is not limited thereto. The buffer tank according to the present disclosure may be applied to the fresh solution buffer tank 6 shown in FIG. 1. In this case, it is possible to shorten a period of time taken until the culture medium heated by the inlet heater 5 is homogenized inside the fresh solution buffer tank 6. Therefore, it is possible to homogenize the culture medium supplied into the passage 104 of the culture container 100 and to improve the quality of the cultured cells.

According to the present disclosure in some embodiments, it is possible to shorten a period of time taken from when a culture medium is injected till when the culture medium is homogenized, which improves the quality of cultured cells.

The present disclosure is not limited to the above-described embodiment and modifications as they stand. At the implementation stage, the constituent elements may be modified and embodied without departing from the present disclosure. In addition, various embodiments may be made by appropriately combining the constituent elements disclosed in the embodiment and modifications described above. Some constituent elements may be deleted from all the constituent elements shown in the embodiment and the modifications. In addition, the constituent elements according to different embodiments and modifications may be appropriately combined.

## Claims

1. A buffer tank (30) for storing a culture medium for cell culture, comprising:
a tank main body (32) including a cylindrical inner surface (33) extending in a vertical direction, an inverted conical storage bottom surface (34) formed below the cylindrical inner surface (33), and a storage space (31) defined by the cylindrical inner surface (33) and the inverted conical storage bottom surface (34) and configured to store the culture medium;
an injection portion (40) configured to inject the culture medium from the inverted conical storage bottom surface (34) of the tank main body (32) into the storage space (31); and
a discharge portion (44) configured to discharge the culture medium stored in the storage space (31),
wherein the injection portion (40) is configured to inject the culture medium toward a central axis line (X) of the cylindrical inner surface (33) of the tank main body (32) and obliquely upward with respect to the central axis line (X).

2. The buffer tank (30) of Claim 1, wherein, when viewing the inverted conical storage bottom surface (34) with reference to a pair of first and second generatrices (34a) in a cross section including the central axis line (X) and the injection portion (40), the injection portion (40) is configured to inject the culture medium in a direction parallel to the first generatrix (34a) existing at a side opposite to the second generatrix (34a) existing at a side where the injection portion (40) is installed.

3. The buffer tank (30) of Claim 1 or 2, wherein the injection portion (40) includes an injection path (41) penetrating the tank main body (32) and an injection opening (42) formed at a downstream end portion of the injection path (41), and
the injection path (41) extends along a plane including the central axis line (X) and the injection opening (42).

4. The buffer tank (30) of any one of Claims 1 to 3, wherein the discharge portion (44) includes a discharge opening (45) formed at a lowermost position of the inverted conical storage bottom surface (34) of the tank main body (32).

5. The buffer tank (30) of any one of Claims 1 to 4, further comprising:
a liquid level sensor (62) configured to detect a liquid level of the culture medium stored in the storage space (31).

6. The buffer tank (30) of any one of Claims 1 to 5, further comprising:
an analyzer (50) configured to analyze components of the culture medium stored in the storage space (31).

7. The buffer tank (30) of Claim 6, wherein the analyzer (50) includes at least one of a pH measurement sensor (51) configured to measure a pH of the culture medium, an oxygen concentration sensor (52) configured to measure a dissolved oxygen concentration in the culture medium, and a carbon dioxide concentration sensor (53) configured to measure a carbon dioxide concentration in the culture medium.

8. The buffer tank (30) of any one of Claims 1 to 7, further comprising:
a heating part (63) installed around the tank main body (32) and configured to heat the tank main body (32).

9. A culture system (1), comprising:
a container holding part (3) configured to hold a culture container (100);
a culture medium analysis part (4) configured to analyze a culture medium discharged from the culture container (100) held by the container holding part (3); and
the buffer tank (30) of any one of Claims 1 to 8 installed between the container holding part (3) and the culture medium analysis part (4) and configured to store the culture medium discharged from the culture container (100) held by the container holding part (3).

10. The culture system (1) of Claim 9, further comprising:
a circulation line (70) configured to circulate the culture medium stored in the storage space (31) of the buffer tank (30).

11. A culture system (1), comprising:
a culture medium supply source (2) configured to supply a fresh culture medium;
a container holding part (3) configured to hold a culture container (100); and
the buffer tank (30) of any one of Claims 1 to 8 installed between the culture medium supply source (2) and the container holding part (3) and configured to store the fresh culture medium supplied from the culture medium supply source (2).
